(19) Europäisches Patentamt
European Patent Office
Office européen des brevets

(11) EP 0 802 261 B1

(12) EUROPÄISCHE PATENTSCHRIFT

(45) Veröffentlichungstag und Bekanntmachung des Hinweises auf die Patenterteilung:
**08.08.2001 Patentblatt 2001/32**

(51) Int Cl.[7]: **C12P 23/00**

(21) Anmeldenummer: **97105918.3**

(22) Anmeldetag: **10.04.1997**

(54) **Enzymatische Acylierung eines Retinolderivates**

Enzymatic acylation of a retinol derivative

Acylation enzymatique d'un dérivé du rétinol

(84) Benannte Vertragsstaaten:
**AT BE CH DE DK ES FR GB IT LI NL**

(30) Priorität: **18.04.1996 EP 96106086**
**20.02.1997 EP 97102768**

(43) Veröffentlichungstag der Anmeldung:
**22.10.1997 Patentblatt 1997/43**

(73) Patentinhaber: **F. HOFFMANN-LA ROCHE AG**
**4070 Basel (CH)**

(72) Erfinder:
- **Orsat, Bernard**
  **4123 Allschwil (CH)**
- **Spurr, Paul**
  **4125 Riehen (CH)**
- **Wirz, Beat**
  **4153 Reinach (CH)**

(74) Vertreter: **Kellenberger, Marcus, Dr. et al**
**F.Hoffmann-La Roche AG**
**Patent Department (PLP),**
**124 Grenzacherstrasse**
**4070 Basel (CH)**

(56) Entgegenhaltungen:
- **J. LIPID RES., Bd. 29, Nr. 12, 1988, Seiten 1693-1697, XP000654926 O'CONNOR J. UND YAGHI B.: "A rapid and sensitive separation of retinol and retinyl palmitate using a small, disposable bonded-phase column: kinetic applications"**
- **TETRAHEDRON LETT., Bd. 31, Nr. 24, 1990, Seiten 3405-3408, XP000654907 RAMASWAMY S. ET AL.: "Porcine pancreatic lipase mediated selective acylation of primary alcohols in organic solvents"**
- **TETRAHEDRON: ASYMMETRY, Bd. 4, Nr. 5, 1993, Seiten 757-760, XP000652320 AKITA H. ET AL.: "Total synthesis of (-)-Oudemansin X based on enzymatic resolution using immobilized lipase"**
- **BIOCHEMISTRY, Bd. 29, Nr. 41, 1990, Seiten 9690-9697, XP000654919 CANADA F.J. ET AL.: "Substrate specifities and mechanism in the enzymatic processing of vitamin A into 11-cis-retinol"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 802 261 B1

Printed by Jouve, 75001 PARIS (FR)

## Beschreibung

Die vorliegende Erfindung betrifft ein neues Verfahren zur Herstellung von (11Z,13Z)-7,10-Dihydro-10-hydroxy-retinyl-acylaten [(2Z,4Z,7E)-Carbonsäure-3,7-dimethyl-6-hydroxy-9-[2',2',6'-trimethyl-cyclohex-6'-en-1'-yl]nona-2,4,7-trienylester] der Formel

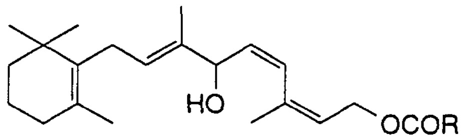

I

worin R eine $C_{1-23}$-Alkylgruppe oder $C_{2-23}$-Alkenylgruppe (wie diese nachträglich näher erläutert wird) bedeutet, durch Enzym-katalysierte, selektive Monoacylierung von (11Z,13Z)-7,10-Dihydro-10-hydroxy-retinol [(2Z,4Z,7E)-3,7-Dimethyl-9-[2',2',6'-trimethylcyclohex-6'-en-1'-yl]nona-2,4,7-trien-1,6-diol] der Formel

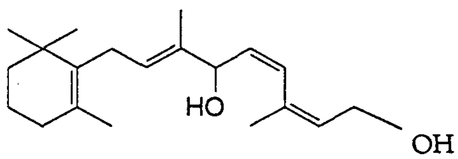

II

(11Z,13Z)-7,10-Dihydro-10-hydroxy-retinyl-acylate sind Ausgangsmaterialien zur Herstellung der entsprechenden Vitamin A-acylate, und zwar durch Wasserabspaltung unter gleichzeitiger cis-trans-Isomerisierung, was auf an sich bekannte Weise erfolgen kann. Mit den üblichen Standard-Veresterungsmethoden erhält man beispielsweise durch Acetylierung von (11Z,13Z)-7,10-Dihydro-10-hydroxy-retinol neben dem erwünschten und besonders wichtigen (11Z,13Z)-7,10-Dihydro-10-hydroxyretinyl-acetat jedoch in unterschiedlichen Mengen auch das als Nebenprodukt anfallende (11Z,13Z)-7,10-Dihydro-10-acetoxy-retinyl-acetat [(2Z,4Z,7E)-Essigsäure-6-acetoxy-3,7-dimethyl-9-[2',2',6'-trimethyl-cyclohex-6'-en-1'-yl]nona-2,4,7-trienylester] der Formel

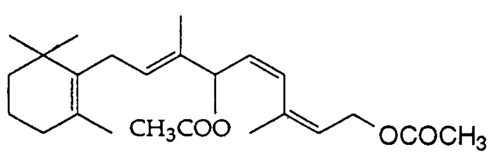

III

Das (11Z,13Z)-7,10-Dihydro-10-acetoxy-retinyl-acetat der Formel III ist unter den Bedingungen der katalytischen Dehydratisierung inert und stört somit nicht nur bei der Aufarbeitung von (11Z,13Z)-7,10-Dihydro-10-hydroxy-retinyl-acetat (der Formel I, in der R Methyl bedeutet) sondern auch bei der Aufarbeitung von Vitamin A-acetat. Deshalb ist ein Verfahren nicht nur für die selektive Monoacetylierung, sondern generell auch für die selektive Monoacylierung von (11Z,13Z)-7,10-Dihydro-10-hydroxy-retinol der Formel II von grossem technischem Interesse.

Der vorliegenden Erfindung liegt nun die Aufgabe zugrunde, ein Verfahren zur Herstellung von (11Z,13Z)-7,10-Dihydro-10-hydroxy-retinylacylaten der Formel I durch Veresterung von (11Z,13Z)-7,10-Dihydro-10-hydroxy-retinol bereitzustellen, das die Nachteile der vorbekannten Arbeitsweisen (z.B. unerwünschte Bildung von (11Z,13Z)-7,10-Dihydro-10-acetoxy-retinyl-acetat der Formel III) nicht aufweist. Dazu ist es notwendig, dass die Enzym-katalysierte Reaktion äusserst selektiv und in hohen Ausbeuten verläuft und dass das Enzym die katalytische Wirkung bereits in geringen Mengen entfaltet, sich leicht abtrennen und mehrfach wiederverwenden lässt.

Im Rahmen der vorliegenden Erfindung wurde diese Aufgabe dadurch gelöst, dass die Acylierung von (11Z,13Z)-7,10-Dihydro-10-hydroxy-retinol der Formel II in Gegenwart einer Lipase (Enzymklasse EC 3.1.1.3) und unter ganz spezifischen Vor- und Reaktionsbedingungen durchgeführt wird.

Demnach betrifft die vorliegende Erfindung ein Verfahren zur Herstellung eines (11Z,13Z)-7,10-Dihydro-10-hydroxy-retinyl-acylats der oben angegebenen Formel I, worin R eine $C_{1-23}$-Alkylgruppe oder eine 1 bis 3 Doppelbindungen aufweisende $C_{2-23}$-Alkenylgruppe bedeutet, welches dadurch gekennzeichnet ist, dass (11Z,13Z)-7,10-Dihydro-10-hydroxy-retinol der ebenfalls oben angegebenen Formel II in einem organischen Lösungsmittel in Gegenwart eines Acylierungsmittels, welches ein Alkyl- oder Alkenylacylat ist, mit einer in Suspension vorliegenden Lipase selektiv monoacyliert wird.

Die selektive Acylierung primärer Hydroxygruppen neben sekundären in Gegenwart von Lipasen ist aus der Literatur bekannt. So wird beispielsweise gemäss J. Org. Chem. 55, 2366-2369 (1990) die Acylierung von Amphenicolen, d.h. von phenylsubstituierten kurzkettigen aliphatischen Diolen, durch Veresterung mit einem Trifluorethylacylat oder einem cyclischen Anhydrid unter Verwendung einer aus *Pseudomonas cyclopium* isolierten Lipase durchgeführt, wobei die Ausbeute 83% bzw. 64% beträgt, ohne dass die Reinheit angegeben ist.

In Tetrahedron: Asymmetry 4, 757-760 (1993) ist die Racematspaltung von 2-Methyl-5-(4-methoxyphenyl)-pentan-1,3-diol durch Veresterung mit Vinylacetat in Gegenwart von immobilisierter Lipase PS beschrieben, wobei die Ausbeute an monoacyliertem Produkt 66% und die optische Reinheit 42% e.e. beträgt.

In Appl. Biochem. Biotechnol. 11, 401-407 (1985) wurde eine Serie von 1,2- und 1,3-Diolen (gelöst in Ethylacylaten) mit Lipase aus Schweinepankreas in Ausbeuten von ≤97% acyliert.

Gemäss J. Chem. Soc., Chem. Commun., 1989, 1535-1536 wurde 2-Ethylhexan-1,3-diol mit Lipase aus Schweinepankreas in 60%iger Ausbeute acyliert.

Tetrahedron Lett. 31, 3405-3408 (1990) beschreibt die selektive Acylierung von aliphatischen 1,n-Diolen mit Anhydriden in Gegenwart von Lipase aus Schweinepankreas, wobei die Selektivitäten ≤98% und die Ausbeuten ≤95% betrugen.

In Ind. J. Chem. 32, 30-34 (1993) ist die selektive Acylierung (98% Selektivität) von 1,5-Hexandiol mit n-Decansäure mit Lipase aus *Chromobacterium viscosum* beschrieben.

In J. Lipid Research 29, 1693 - 1697 ist im Zusammenhang mit einer Methode zur chromatographischen Trennung von Retinol und dessen Palmitat voneinander eine Enzym-katalysierte Synthese von Retinylpalmitat aus Retinol und Palmitinsäure beschrieben, wobei es sich beim Katalysator um Gallensalz-stimulierte Muttermilch-Lipase handelt. Die Veresterung erfolgt in der Flüssigphase, in welcher auch Rinderserumalbumin und Natriumtaurocholat vorhanden sind.

Alle diese vorbekannten Verfahren weisen gewichtige Nachteile auf: so liefern zwar alle diese Verfahren die erwünschten Produkte, doch lässt die Selektivität und damit die Reinheit und/oder die Ausbeute zu wünschen übrig. Darüber hinaus ist in keiner dieser Literaturstellen von einer mehrmaligen Verwendung der eingesetzten Lipase, also von der Stabilität der Lipase, die Rede, geschweige denn von einer gleichzeitigen Substratreinigung.

Die Acylierung von (11Z,13Z)-7,10-Dihydro-10-hydroxy-retinol der Formel II erfolgt wie bereits erwähnt in einem - vorzugsweise nahezu wasserfreien - organischen Lösungsmittel in Gegenwart eines Acylierungsmittels ,welches ein Alkyl - oder Alkenylacylat ist, mit einer in Suspension vorliegenden Lipase.

Für den Zweck der vorliegenden Erfindung geeignete Lipasen sind solche der Enzymklasse EC 3.1.1.3, welche gute Aktivität und Selektivität zeigen, insbesondere Lipase PL aus *Alcaligenes sp*., deren immobilisierte Formen Lipase PLC und Lipase PLG, und Lipase MY-30 aus *Candida cylindracea* (umbenannt *Candida rugosa*) (Firma Meito Sangyo, Tokyo, Japan), Lipozyme® IM-20 aus *Mucor miehei* (umbenannt *Rhizomucor miehei*) (Firma Novo Nordisk, Bagsvaerd, Dänemark), Lipase CE-5 aus *Humicola lanuginosa* und Lipase G aus *Penicillium cyclopium* (beide Firma Amano Pharmaceutical Co. Ltd., Nagoya, Japan) sowie Chirazyme® L-2 aus *Candida antarctica* (Firma Boehringer Mannheim GmbH, Deutschland; ehemals Novozym® SP 435 der Firma Novo Nordisk). Besonders bevorzugt sind Lipase PL, Lipase PLC, Lipase PLG, Lipozyme® IM-20 und Chirazyme® L-2, ganz besonders bevorzugt Lipase PL, Lipase PLC, Lipase PLG und Chirazyme® L-2.

Unter dem Ausdruck "$C_{1-23}$-Alkylgruppe" oder "1 bis 3 Doppelbindungen aufweisende $C_{2-23}$-Alkenylgruppe" (R) sind je nach Anzahl der Kohlenstoffatome sowohl geradkettige als auch verzweigte Alkyl- bzw. Alkenylgruppen zu verstehen. Beispiele der $C_{1-23}$-Alkylgruppen sind Methyl, Ethyl, Propyl, Pentyl, Heptyl, Undecyl, Pentadecyl und Heptadecyl, bzw. der $C_{2-23}$-Alkenylgruppen 8-Heptadecenyl und Heptadeca-8,11-dienyl. Die entsprechenden Alkanoyl- und Alkenoylgruppen (RCO) sind Acetyl, Propionyl, Butyryl, Lauryl, Caproyl, Capryl, Palmitoyl und Stearoyl bzw. Oleoyl und Linolyl. Eine besonders bevorzugte Bedeutung von R ist Methyl, wobei es sich bei dem erfindungsgemässen Verfahren um eine Enzym-katalysierte, selektive Monoacetylierung handelt.

Im Batch-Verfahren wird die Lipase, welche in kommerziell erhältlicher Form als Pulver, Körner oder kleine Kugeln vorliegt, in bis zu 20 Gewichts-(Gew.-)% (Gew./Gew. bezogen auf (11Z,13Z)-7,10-Dihydro-10-hydroxy-retinol) eingesetzt, vorzugsweise liegt jedoch der Anteil Lipase zwischen etwa 0,1 Gew.-% und etwa 10 Gew.-%, besonders bevorzugt zwischen etwa 1 Gew.-% und etwa 5 Gew.-%.

Als organische Lösungsmittel, welche sich für den Zweck der vorliegenden Erfindung eignen, können genannt werden: aliphatische Kohlenwasserstoffe mit 5-8 Kohlenstoffatomen, wie Hexan und Heptan; alicyclische Kohlenwasserstoffe mit 6-10 Kohlenstoffatomen, wie Cyclohexan, Methylcyclohexan und Decalin; chlorierte aliphatische Kohlenwasserstoffe, wie Methylenchlorid, Chloroform und Tetrachlorkohlenstoff; nitrosubstituierte aliphatische Kohlenwasserstoffe, wie Nitromethan; aromatische Kohlenwasserstoffe, wie Toluen und Xylen; aliphatische Ether, wie 1,2-Dimethoxyethan, Diethylether, Diisopropylether und tert.-Butylmethylether; cyclische Ether, wie Tetrahydrofuran, Methylfuran und 1,4-Dioxan; aliphatische Ester, wie Trimethylorthoformiat, Ethylacetat, Butylacetat, Vinylacetat, Vinylpropionat und Isopropenylacetat; aliphatische Ketone, wie Aceton; aliphatische Nitrile, wie Acetonitril; aliphatische Amine, wie Triethylamin; aliphatische Acetale, wie Formaldehyd-dimethylacetal; und Gemische solcher Lösungsmittel. Bevorzugt sind Hexan, Cyclohexan, Methylenchlorid, Tetrachlorkohlenstoff, Toluen, Diisopropylether, Vinylacetat, Vinylpropionat, Tetrahydrofuran, Methylfuran und Formaldehyd-dimethylacetal, insbesondere Methylenchlorid, Diisopropylether, Vinylacetat, Vinylpropionat, Tetrahydrofuran, Formaldehyd-dimethylacetal und Gemische dieser Lösungsmittel.

Als Acylierungsmittel können die verschiedenen gängigen Alkyl- und Alkenylacylate, wie Methylacetat, Ethylacetat, Butylacetat, Vinylacetat, Allylacetat, Isopropenylacetat, Ethylpropionat, Ethylbutyrat und Vinylpropionat sowie Ester längerkettiger Fettsäuren, z.B. Vinyllaurat, eingesetzt werden. Bevorzugt wird zur Acetylierung Ethylacetat, Butylacetat oder Vinylacetat, besonders bevorzugt Vinylacetat, verwendet. Für die Herstellung des Propionats (R = Ethyl) ist Vinylpropionat bevorzugt, und auch für die Herstellung längerkettiger Acylate sind die Vinyl-Ester der entsprechenden Fettsäuren bevorzugt. Die Menge an eingesetztem Acylierungsmittel kann zwischen 1 Mol-Aequivalent und einem mehrfachen Ueberschuss liegen; das Acylierungsmittel wird insbesondere dann im Ueberschuss verwendet, wenn es gleichzeitig als Lösungsmittel dient, was bei den Alkyl- und Alkenylacylaten der Fall sein kann.

Die Konzentration an (11Z,13Z)-7,10-Dihydro-10-hydroxy-retinol beträgt zweckmässig 10% bis 50%, vorzugsweise 20% bis 40% [in Gewicht/Volumen (G/V) ausgedruckt]: Die Löslichkeit des Edukts wird dabei durch die Wahl des Lösungsmittels bzw. Lösungsmittelgemisches und/oder der Temperatur gesteuert. So liegt die Reaktionstemperatur vorteilhafterweise zwischen etwa 10°C und der Rückflusstemperatur des Reaktionsgemisches, vorzugsweise zwischen der Raumtemperatur und etwa 90°C, besonders bevorzugt zwischen der Raumtemperatur und etwa 60°C. Im Batch-Verfahren braucht das (11Z,13Z)-7,10-Dihydro-10-hydroxy-retinol am Anfang der Reaktion nicht gelöst zu sein; vielmehr kann am Anfang der Reaktion auch eine Suspension vorliegen.

Um die Zugänglichkeit zur Lipase und deren Wiederverwendung zu verbessern, kann sie auf verschiedenen Trägermaterialien immobilisiert werden. Diese Immobilisierung kann kovalent oder nicht-kovalent erfolgen, vorzugsweise nicht-kovalent, durch einfache Adsorption auf ein geeignetes Trägermaterial mit grosser Oberfläche. Da Lipase und Trägermaterial in organischen Lösungsmitteln unlöslich sind, erfolgt während der Reaktion keine messbare Desorption. Als Trägermaterialien eignen sich viele der üblichen, billigen Filterhilfstoffe, Adsorbentien, Ionenaustauscher und Chromatographiematerialien, wie Florisil® , Diatomeenerde, Bentonite, Cellulose, Molekularsieb, Amberlite® , Amberlyst® , Kieselgel oder Aluminiumoxid, und dergleichen, aber auch andere billige Materialien mit grossen Oberflächen, wie Sand, gesintertes Glas oder Hohlfasern und dergleichen. Bevorzugt ist die Verwendung von Diatomeenerde und Seesand. Alternativ können auch kommerziell erhältliche, bereits immobilisierte Lipasepräparate eingesetzt werden, beispielsweise die Lipasepräparate der Firma Meito Sangyo bzw. Boehringer Mannheim GmbH:

- Lipase PLC: Lipase PL immobilisiert auf Diatomeenerde;
- Lipase PLG: Lipase PL immobilisiert auf granulierter Diatomeenerde;
- Chirazyme® L-2 (ehemals Novozym® SP 435): Lipase aus *Candida antarctica*, immobilisiert auf makroporösem Polyacryl.

Erwünschtenfalls kann die Immobilisierung der Lipase auch in Gegenwart eines "Gallensalzes" erfolgen (Coimmobilisierung), wodurch die Aktivität teilweise gesteigert werden kann (Aktivator). Geeignete Gallensalze sind z.B. Natriumcholat und Natriumdeoxycholat.

Um die Gefahr chemischer Nebenreaktionen praktisch von vornherein auszuschliessen, wird die Umsetzung mit der Lipase zweckmässig unter einer Inertgasatmosphäre, z.B. Stickstoff oder Argon, und unter Lichtausschluss und/oder in der Gegenwart eines Radikalfängers, z.B. Hydrochinon oder 2,6-Di(tert.butyl)-p-cresol, durchgeführt.

Der Katalysator kann im Batch-Verfahren nach einem Versuch abfiltriert und wiederverwendet werden. Für die Stabilität und Aktivität der Lipase spielt unter anderem der Wassergehalt der Reaktionslösung - und damit der Lipase - eine Rolle. Die Zugabe einer geringen Menge Wasser (<0.2% der Reaktionslösung) hat dabei einen positiven Einfluss auf die Lipaseaktivität; noch besser wirken schwach basische Lösungen, wie Ammoniumbicarbonat- oder Ammoniumhydroxid-Lösungen, oder organische Basen, z.B. Triethylamin oder Ethyldiisopropylamin. Die Zugabe des Wassers kann auch nur periodisch oder im Sinne eines Aequilibrierungsschrittes erfolgen.

Für die effiziente Wiederverwendung der Lipase, welche für die Wirtschaftlichkeit des Verfahrens äussertst wichtig ist, ist auch die Reinheit des Edukts entscheidend: Durch eine entsprechende Reinigung des Edukts, welches aus der Vorstufe u.a. noch Schwermetalle enthält, kann die Langzeitstabilität des Lipasepräparates beträchtlich verbessert werden. Als einfache und wirkungsvolle Reinigungsverfahren haben sich das Filtrieren über verschiedene Hilfsstoffe, wie Diatomeenerde, Kieselgel, Ethylendiamin-tetraessigsäure (EDTA)-Salze und Aluminiumoxid, und das Waschen mit wässriger EDTA-Lösung bei einem pH-Wert von etwa 8 erwiesen, mit deren Hilfe u.a. der Schwermetallgehalt des Edukts gesenkt und die Wirksamkeit des Lipasepräparates verbessert werden kann.

Das erfindungsgemässe Verfahren kann als wiederholtes Batch-Verfahren oder als kontinuierliches Verfahren durchgeführt werden, und zwar unter Verwendung gängiger Reaktortypen, wie beispielsweise Festbettreaktor, Schlauchreaktor, Faserreaktor, Umlaufreaktor, Wirbelschichtreaktor (mit Zwischenboden) oder Slurry-Reaktor.

Einen weiteren Aspekt der vorliegenden Erfindung stellt die oben erwähnte Verwendung eines erfindungsgemässen erhaltenen (11Z,13Z)-7,10-Dihydro-10-hydroxy-retinyl-acylats, vorzugsweise des Acetats, zur Herstellung des entsprechenden Vitamin A-acylats bzw. des Vitamin A-acetats dar.

Die folgenden Beispiele zur Herstellung von verschiedenen (11Z,13Z)-7,10-Dihydro-10-hydroxy-retinyl-acylaten durch Enzym-katalysierte, selektive Monoacylierung von (11Z,13Z)-7,10-Dihydro-10-hydroxy-retinol zeigen vorteilhafte Ausführungsformen des erfindungsgemässen Verfahrens auf, sollen jedoch keinerlei Einschänkung darstellen. Alle Temperaturen sind in Celsiusgraden angegeben.

Beispiel 1

10,0 g (32,8 mmol) (11Z,13Z)-7,10-Dihydro-10-hydroxy-retinol und 10 mg 2,6-Di-(tert.butyl)-p-cresol (BHT) wurden in einem Gemisch von 30 ml Toluen und 5 ml Vinylacetat (54,1 mmol) gelöst. Die Reaktion wurde durch Zugabe von 500 mg Lipase PLC (Meito Sangyo) gestartet, und die Suspension 17 Stunden bei Raumtemperatur auf einem Roller leicht gerührt. Die Lipase wurde danach abfiltriert und mit Toluen gewaschen, und das Filtrat eingedampft. Nach eintägigem Trocknen unter Hochvakuum erhielt man 11,4 g (33 mmol) (11Z,13Z)-7,10-Dihydro-10-hydroxy-retinyl-acetat in einer Ausbeute von 100,6% und einer Reinheit von >99% [gemäss überkritischer (supercritical) Flüssigchromatographie (SFC)-Flächenprozent].

Beispiel 2

10,0 g (32,8 mmol) (11Z,13Z)-7,10-Dihydro-10-hydroxy-retinol wurden in ein Gemisch von 6,8 ml Toluen und 3,2 ml (34,6 mmol) Vinylacetat aufgenommen. Die Reaktion wurde durch Zugabe von 500 mg Lipase PLC (Meito Sangyo) gestartet, und das Reaktionsgemisch 16 Stunden bei Raumtemperatur leicht gerührt. Die Lipase wurde danach abfiltriert und mit Toluen gewaschen, und das Filtrat eingedampft. Nach eintägigem Trocknen unter Hochvakuum und Rühren erhielt man 11,23 g (32,4 mmol) (11Z,13Z)-7,10-Dihydro-10-hydroxy-retinyl-acetat in einer Ausbeute von 98,8% und einer Reinheit von 97,2% (SFC-Flächenprozent).

Beispiel 3

20,0 g (65,7 mmol) (11Z,13Z)-7,10-Dihydro-10-hydroxy-retinol und 20 mg BHT wurden in 56 ml Toluen, 56 ml tert.Butylmethylether (TBME) und 7,5 ml (81,2 mmol) Vinylacetat gelöst. Die Reaktion wurde durch Zugabe von 1.0 g Lipase PLC (Meito Sangyo) gestartet, und die Suspension 16 Stunden bei 40° leicht gerührt. Die Lipase wurde danach abfiltriert und mit Toluen gewaschen, und das Filtrat eingedampft. Nach eintägigem Trocknen unter Hochvakuum erhielt man 23,1 g (66,7 mmol) (11Z,13Z)-7,10-Dihydro-10-hydroxy-retinyl-acetat in einer Ausbeute von 101,5% und einer Reinheit von >99% (SFC-Flächenprozent).

Beispiel 4

*A) Immobilisierung auf diversen Trägermaterialien:* 2,0 ml Lipase PL-Lösung (25 mg/ml bidestilliertem Wasser) wurden zu 2,5 ml Trägermaterial gegeben, und die erhaltene Suspension bei Raumtemperatur schonend gerührt. Der gleichmässig benetzte Träger wurde durch allmähliches, vorsichtiges Erhöhen des Vakuums (Schaumbildung) getrocknet. Der Träger wurde schliesslich 2 Tage am Hochvakuum getrocknet und, wie nachfolgend beschrieben, eingesetzt.

*B) Immobilisierung auf porösen Glaskugeln:* 2,0 ml Lipase PL-Lösung (20 mg/ml bidestilliertem Wasser), wurden zu 2,0 g Siran Carrier Sikug 041/02/120/A (poröses, gesintertes Glas der Firma Schott Glaswerke, Mainz, Deutschland) gegeben, und die Suspension, wie oben unter A) beschrieben, getrocknet.

Zu den wie oben beschrieben erhaltenen, getrockneten Lipasepräparaten wurden jeweils 8,0 ml eines 2:2:1-Gemisches von Hexan, tert.Butylmethylether (TBME) und Vinylacetat, enthaltend 1,0 g (11Z,13Z)-7,10-Dihydro-10-hydroxy-retinol, gegeben, und die Reaktionslösung bei Raumtemperatur auf einem Roller leicht gerührt. Nach 6 und 27 Stunden wurden je 25 μl-Proben für die HPLC-Analytik entnommen. Die Ergebnisse sind in der nachfolgenden Tabelle 1, in der A für (11Z,13Z)-7,10-Dihydro-10-hydroxyretinol und B für (11Z,13Z)-7,10-Dihydro-10-hydroxy-retinyl-acetat stehen, zusammengefasst.

Tabelle 1

| | | HPLC-Flächenprozent | | | |
|---|---|---|---|---|---|
| # | Trägermaterial | A | | B | |
| | | nach 6 Std. | 27 Std. | nach 6 Std. | 27 Std. |
| 1 | Kieselgel 60 (0,2-0,5 mm; Merck) | | 38,2 | | 61,8 |
| 2 | Florisil® | | 43,9 | | 56,1 |
| 3 | Seesand (40-100 mesh; Fluka 84880) | 40,4 | 4,9 | 59,6 | 95,1 |
| 4 | Molekularsieb (3 Å; Merck) | | 35,9 | | 64,1 |
| 5 | Avicel® (mikrokristalline Cellulose) | | 29,0 | | 71,0 |
| 6 | DICALITE® Speedex (Diatomeenerde) | 34,7 | 3,8 | 65,3 | 96,2 |
| 7 | Hyflo Super Cel® (Kieselgur, Fluka 56678) | | 36,8 | | 63,2 |
| 8 | Clarcel Typ Die/B (Diatomeenerde der Firma CECA, Paris, Frankreich) | 41,1 | 6,5 | 58,9 | 93,5 |
| 9 | Bentonite (Fluka 11957) | | 74,8 | | 25,2 |
| 10 | Bentonite (Fluka 11959) | | 95,2 | | 4,8 |
| 11 | Aluminiumoxid neutral I (Camag 507-C der Firma Camag, Muttenz, Schweiz) | | 33,5 | | 66,5 |
| 12 | Aluminiumoxid basisch I (Camag 5016-A) | | 31,1 | | 68,9 |
| 13 | Amberlite® XAD-7 (20-50 mesh) | | 33,1 | | 66,9 |
| 14 | Amberlite® XAD-8 (20-50 mesh) | | 46,2 | | 53,8 |
| 15 | Amberlyst® A-21 (0,4-0,55 mm) | 38,0 | 12,6 | 62,0 | 87,4 |
| 16 | Amberlite® IR-45 | | 21,9 | | 78,1 |
| 17 | Amberlite® IRA-93 | 33,3 | 9,2 | 66,7 | 90,8 |
| 18 | Glaskügelchen (0,5 mm) | | 70,1 | | 29,9 |
| 19 | Siran Carrier Sikug 041/02/120/A | | 34,1 | | 65,9 |

Beispiel 5

8,0 ml einer Lösung von 1,0 g (11Z,13Z)-7,10-Dihydro-10-hydroxy-retinol in einem 2:2:1-Gemisch von Hexan, TBME und Vinylacetat wurden mit jeweils 30 mg Lipase PLC bzw. Lipase PLG versetzt, und die Suspension auf einem Roller unter Argon bei Raumtemperatur im Dunkeln leicht gerührt. Nach 24 Stunden war der Restgehalt an (11Z,13Z)-7,10-Dihydro-10-hydroxyretinol weniger als 1% (HPLC). Bei Verwendung von jeweils 51 mg Lipase PLC bzw. Lipase PLG war nach 24 Stunden kein (11Z,13Z)-7,10-Dihydro-10-hydroxy-retinol mehr nachweisbar.

Beispiel 6

25 mg Lipase PL und 0,5 mg Cholatsalz (Natriumcholat, Natriumdeoxycholat) wurden in 2,0 ml bidestilliertem Wasser gelöst und zu 2,5 g in 6 ml bidestilliertem Wasser aufgeschlämmtem DICALITE® Speedex bzw. Seesand gegeben. Die Suspension wurde wie in Beispiel 4 beschrieben vorsichtig getrocknet und das Trockengut nötigenfalls zerrieben.

Die hergestellten Lipasepräparate wurden wie in Beispiel 5 beschrieben mit 8 ml (11Z,13Z)-7,10-Dihydro-10-hydroxy-retinol-Lösung getestet. Nach jeweils 24 Stunden wurde mittels HPLC analysiert. Die Ergebnisse sind in der nachfolgenden Tabelle 2, in der A für (11Z,13Z)-7,10-Dihydro-10-hydroxyretinol steht, zusammengefasst.

Tabelle 2

| Cholat-Salz | Träger | Restgehalt an A (HPLC-Flächen-%) |
|---|---|---|
| keines | DICALITE® | 9,1 |
| | Seesand | 37,8 |

Tabelle 2 (fortgesetzt)

| Cholat-Salz | Träger | Restgehalt an A (HPLC-Flächen-%) |
|---|---|---|
| Natriumcholat | DICALITE® | 6,5 |
| | Seesand | 31,2 |
| Natriumdeoxycholat | DICALITE® | 5,6 |
| | Seesand | 32,3 |

Beispiel 7

50,0 g (164,2 mmol) (11Z,13Z)-7,10-Dihydro-10-hydroxy-retinol wurden in einem Gemisch von 140 ml Hexan, 140 ml TBME, 70 ml Vinylacetat und 50 mg BHT gelöst. 2,5 g Lipase PLC wurden zugegeben, und die Suspension auf einem Roller bei Raumtemperatur unter Argon und im Dunkeln 24 Stunden leicht gerührt. Dann wurde der Lipasekatalysator abfiltriert, mit Diethylether gewaschen und zur Wiederverwendung getrocknet. Das Filtrat wurde zunächst zusammen mit der Waschlösung und danach zuerst zusammen mit zwei 300 ml-Portionen von Hexan und dann mit 300 ml Pentan eingedampft, um auf diese Weise Spuren gebildeter Essigsäure azeotrop zu entfernen. Nach etwa 16-stündigem Trocknen am Hochvakuum bei 35° wurden 56,73 g (163,7 mmol, 99 %) (11Z,13Z)-7,10-Dihydro-10-hydroxy-retinylacetat als schwach gelbliches Oel erhalten.

Analytik: >99% Reinheit (SFC-Flächenprozent); bestätigt gemäss 250-MHz-$^1$H-NMR ($CDCl_3$), EI-MS (m/e 346), IR(Film) und Mikroanalyse:

| | | |
|---|---|---|
| Ber. : | C 76,26%; | H 9,89%; |
| Gef. : | C 76,07%; | H 9,73%. |

Beispiel 8

Je 8,0 ml einer Lösung von 1,0 g (11Z,13Z)-7,10-Dihydro-10-hydroxyretinol und 1 mg BHT in einem 2:2:1-Gemisch von Hexan, TBME und Vinylacetat wurden zu jeweils 50 mg Lipase PLC gegeben, welche bereits vorher schon viermal (als ein einziger Batch) unter den gleichen Bedingungen eingesetzt und zwischen den einzelnen Ansätzen lediglich mit einem 1:1-Gemisch von Hexan und TBME gewaschen worden war. Zu den einzelnen Proben wurden nun verschiedene wässrige Lösungen zugesetzt, die Reaktionssuspensionen auf einem Roller 17 Stunden leicht gerührt und dann mittels HPLC analysiert. Die Ergebnisse sind in der nachfolgenden Tabelle 3, in der B für (11Z,13Z)-7,10-Dihydro-10-hydroxy-retinyl-acetat steht, zusammengefasst.

Tabelle 3

| Zusatz | gebildetes B (HPLC-Flächen-%) |
|---|---|
| keiner | 6 |
| 10 µl Wasser | 19 |
| 10 µl 0,1 M Ammoniumbicarbonat-Lösung | 28 |

Beispiel 9

A. 1 kg (11Z,13Z)-7,10-Dihydro-10-hydroxy-retinol wurde in 8 l Ethylacetat gelöst und mit zwei 3 l-Portionen einer 50 mmolaren EDTA-Lösung mit einem pH-Wert von 8,0 bei Raumtemperatur gewaschen. Die organische Phase wurde danach über wasserfreiem Natriumsulfat getrocknet und eingedampft, und der Rückstand am Hochvakuum getrocknet.

B. Jeweils 50 g (11Z,13Z)-7,10-Dihydro-10-hydroxy-retinol wurden in 400 ml Ethylacetat gelöst und jeweils über 5 g Aluminiumoxid basisch, DICALITE® Speedex bzw. Kieselgel filtriert. Das Filtrat wurde eingedampft und am Hochvakuum getrocknet.

C. Jeweils 3,5 g der gemäss A. bzw. B. gereinigten (11Z,13Z)-7,10-Dihydro-10-hydroxy-retinol-Proben wurden in

25 ml eines 2:2:1-Gemisches von Toluen, TBME und Vinylacetat gelöst. Von jeder Lösung wurden drei 8,0 ml-Proben entnommen (Dreifachversuch) und jeweils mit 2,5 mg Lipase PLC versetzt. Die Reaktionssuspension wurde 24 Stunden bei Raumtemperatur auf einem Roller leicht gerührt und anschliessend mittels SFC analysiert. Die Ergebnisse sind in der nachfolgenden Tabelle 4, in der A und B für (11Z,13Z)-7,10-Dihydro-10-hydroxy-retinol bzw. (11Z,13Z)-7,10-Dihydro-10-hydroxy-retinyl-acetat stehen, zusammengefasst.

Tabelle 4

| Reinigungsmethode | Gebildetes B (SFC-Flächenprozent; minimal - maximal) | A + B (SFC-Flächenprozent: Mittelwert) |
|---|---|---|
| ungereinigt | 24,3 - 27,9 | 95,0 |
| EDTA | 73,2 - 77,8 | 99,4 |
| Aluminiumoxid basisch | 69,0 - 70,4 | 99,6 |
| DICALITE® Speedex | 68,1 - 73,4 | 99,7 |
| Kieselgel | 51,7 - 55,2 | 99,7 |

Beispiel 10

In einer 2-l-Chromatographiesäule mit Glasfritte wurden 5,0 g Lipase PLC vorgelegt. Dann wurde in dieser Reihenfolge 100,0 g (11Z,13Z)-7,10-Dihydro-10-hydroxy-retinol, 280 ml Hexan, 280 ml TBME, 140 ml Vinylacetat, welches gegebenenfalls 500 µl einer 0,1 M Ammoniumbicarbonat- bzw. einer 1%igen Ammoniumhydroxid-Lösung enthielt, und 100 mg BHT zugegeben und die Säule durch Drehen (Rührmotor) leicht gerührt, wobei die Säule mit dem Auslass nach oben geneigt war, so dass die Fritte von der Lösung nicht berührt wurde. Nach 17-stündiger Inkubation bei Raumtemperatur wurde die Reaktionslösung abgelassen und mittels SFC analysiert. Das zurückgebliebene Lipasepräparat wurde zunächst dreimal mit je 100 ml eines 1:1-Gemisches von Hexan und TBME (ca. je 15 Minuten pro Waschung) und danach mit 50 ml n-Hexan gewaschen und dann wieder für einen neuen Zyklus eingesetzt. Die Ergebnisse sind in der nachfolgenden Tabelle 5, in der A und B für (11Z,13Z)-7,10-Dihydro-10-hydroxy-retinol bzw. (11Z, 13Z)-7,10-Dihydro-10-hydroxy-retinyl-acetat stehen, zusammengefasst.

Tabelle 5

| | gebildetes B (SFC-Flächenprozent; A+B= 100%) | | | |
|---|---|---|---|---|
| | 1. Zyklus | 2. Zyklus | 3. Zyklus | 4. Zyklus |
| A (ungereinigt) | 99,0 | 92,0 | 50,6 | 22,7 |
| A + 0,1 M $NH_4HCO_3$ pro Ansatz | 99,6 | 99,4 | 95,0 | 76,3 |
| A + 1% $NH_4OH$ pro Ansatz (Waschlösung enthält 1‰ V/V 1% $NH_4OH$) | 100 | 99,4 | 97,8 | 88,7 |
| A gereinigt gemäss Beispiel 9 A. | 100 | 100 | 100 | 99,8 |

Beispiel 11

(11Z,13Z)-7,10-Dihydro-10-hydroxy-retinol wurde wie in Beispiel 9 A beschrieben gereinigt und danach wie in Beispiel 10 beschrieben in einem wiederholten Batch-Verfahren eingesetzt, wobei jedoch 3,0 g Lipase PLC, 90,0 g (11Z,13Z)-7,10-Dihydro-10-hydroxy-retinol, 250 ml Toluen, 250 ml TBME und 125 ml Vinylacetat, welches 450 µl 1% Ammoniumhydroxid-Lösung enthielt, verwendet wurden. Die Reaktionszeit betrug 23 Stunden. Zwischen den Zyklen wurde das Lipasepräparat zweimal mit je 100 ml eines 1:1-Gemisches von Toluen und TBME, welcher 1‰ (V/V) einer 1%igen Ammoniumhydroxid-Lösung enthielt, gewaschen, und jeweils nach 4 Zyklen 3 Tage bei Raumtemperatur in Toluen stehen gelassen. Die Ergebnisse sind wie folgt graphisch dargestellt:

Resultate:

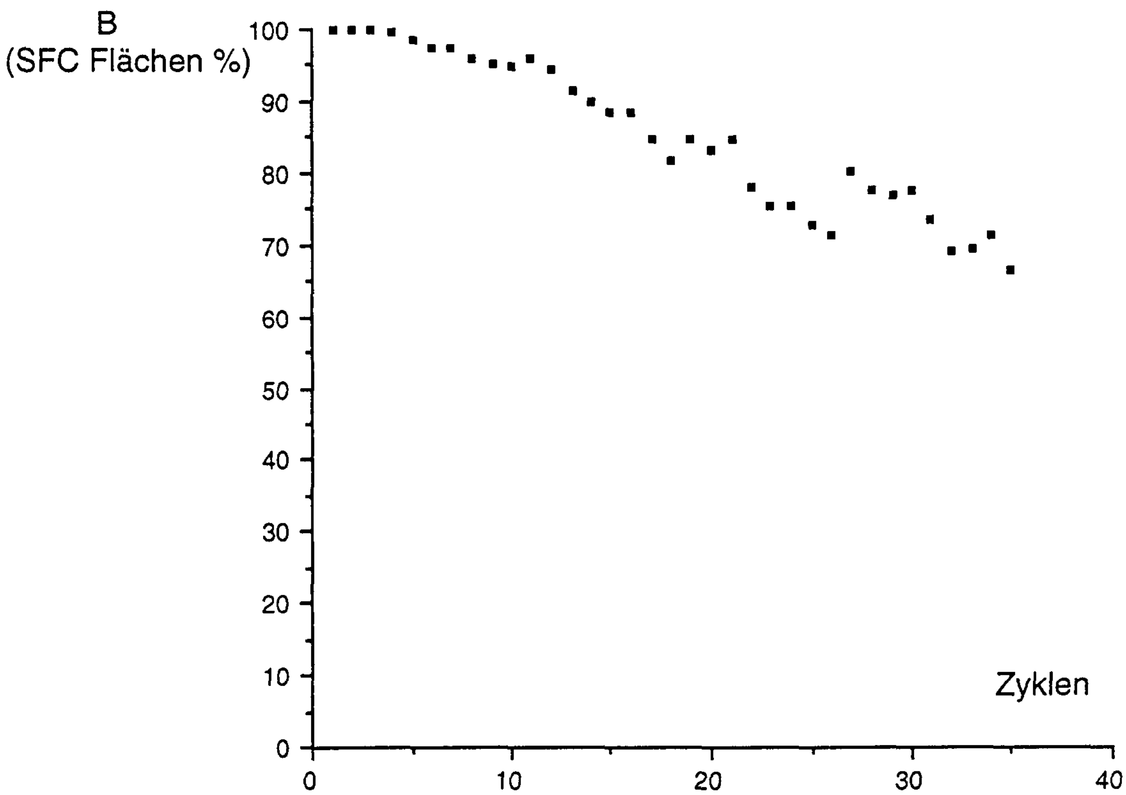


B = (11Z,13Z)-7,10-Dihydro-10-hydroxy-retinylacetat

Beispiel 12

Die Vorgehensweise war wie in Beispiel 11 beschrieben, ausser dass das (11Z,13Z)-7,10-Dihydro-10-hydroxy-retinol wie in Beispiel 9 B beschrieben unter Verwendung von DICALITE® Speedex gereinigt wurde . Die Ergebnisse sind wie folgt graphisch dargestellt:

Resultat:

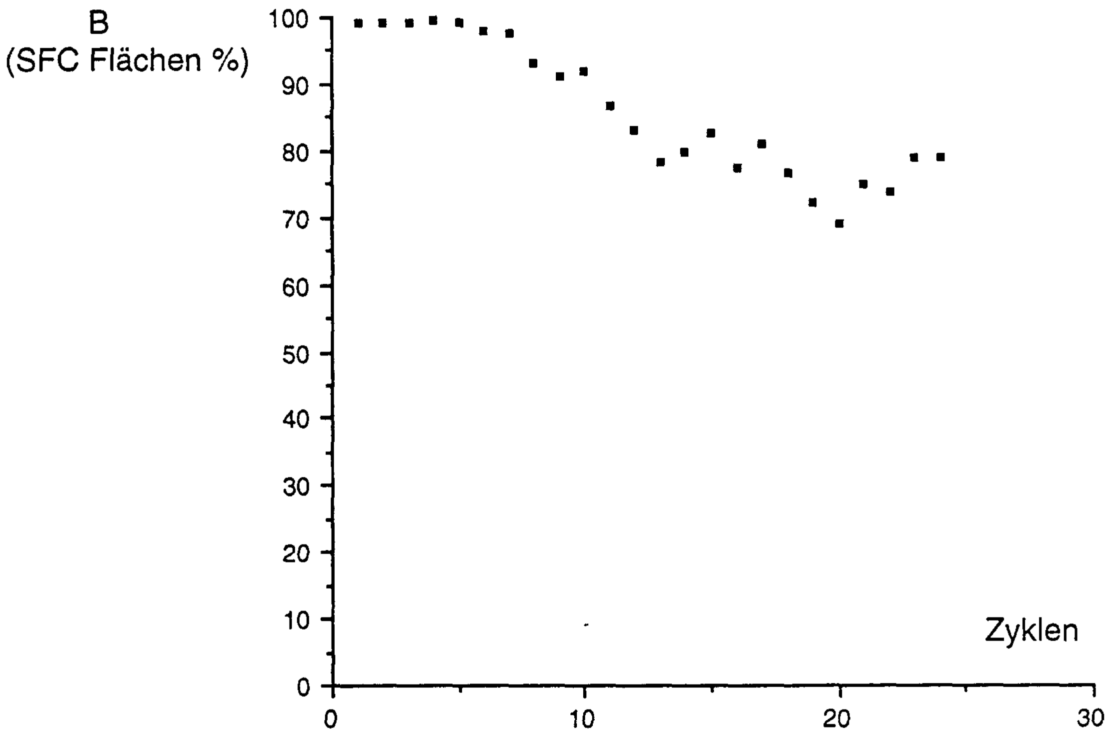


B = (11Z,13Z)-7,10-Dihydro-10-hydroxy-retinylacetat

Beispiel 13

Der Einfluss der Temperatur auf die Reaktion wurde mit einem kontinuierlichen Verfahrensexperiment geprüft. 18,0 g (59,1 mmol, 14,4% G/V) (11Z,13Z)-7,10-Dihydro-10-hydroxy-retinol wurden in einem Gemisch von 100 ml Toluen, welches 300 ppm Wasser enthielt, und 25 ml Vinylacetat gelöst. Das homogene Gemisch wurde über einen Membranfilter [RC60, 1µm, Schleicher & Schuell AG, Dassel, Deutschland (S&S)] filtriert und danach durch eine Kolonne von 10 mm Durchmesser, welche nacheinander mit 7 g DICALITE® Speedex und 600 mg Lipase PLC (Meito Sangyo) gefüllt worden war, mit einer Durchlaufgeschwindigkeit von 0,5 ml/Minute gepumpt. Die Temperatur wurde jeweils zwischen 20 and 50° variiert. Reaktionsproben wurden jeweils entnommen und mittels SFC und HPLC analysiert. Die Ergebnisse sind wie folgt graphisch dargestellt:

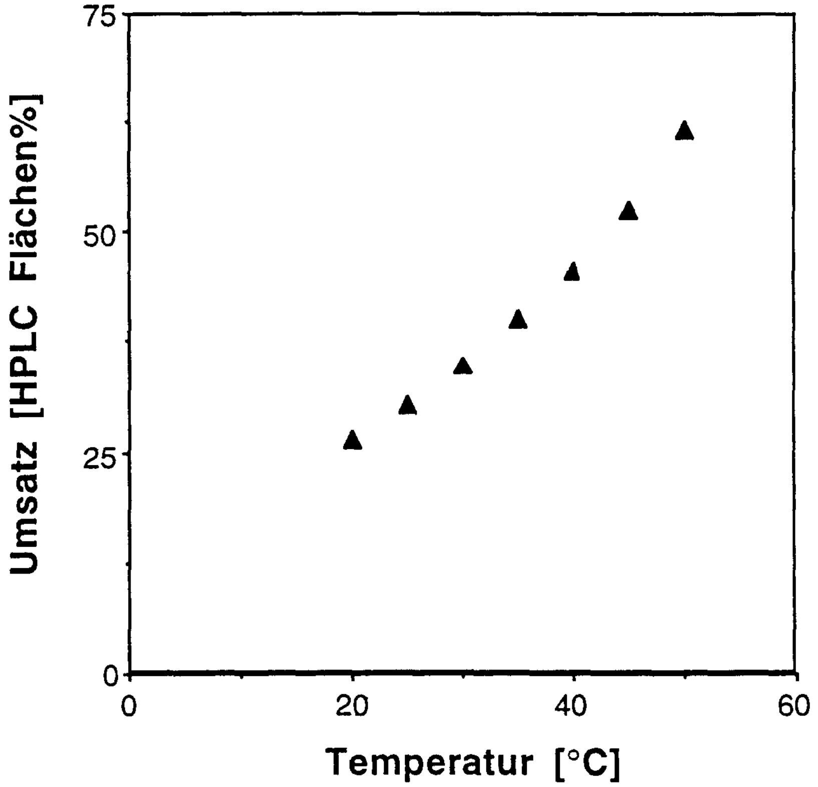


Beispiel 14

Der Einfluss der Temperatur auf die Reaktion wurde ebenfalls mit einem Batch-Verfahrensexperiment geprüft. 48,0 g (157,6 mmol) (11Z,13Z)-7,10-Dihydro-10-hydroxy-retinol wurden in soviel Toluen, welches 20% (G/V) Vinylacetat enthielt, gelöst, dass das erhaltene Gemisch ein Volumen von 240 ml aufwies. Das homogene Gemisch wurde dann über einen Membranfilter (RC60, 1µm, S&S) filtriert und unter Rühren (100-110 Umdrehungen/ Minute) bei 50° gehalten. Die Reaktion wurde durch Zugabe von 3,0 g Lipase PLC (Meito Sangyo) gestartet und mittels HPLC überwacht. Die Ergebnisse sind wie folgt tabellarisch (Tabelle 6) und graphisch angegeben:

Tabelle 6

| Temperatur (°C) | Reaktionszeit (Min.) | Ausbeute (% HPLC) |
|---|---|---|
| Raumtemp. | 1830 | 73,9 |
| 50 | 780 | 99,9 |

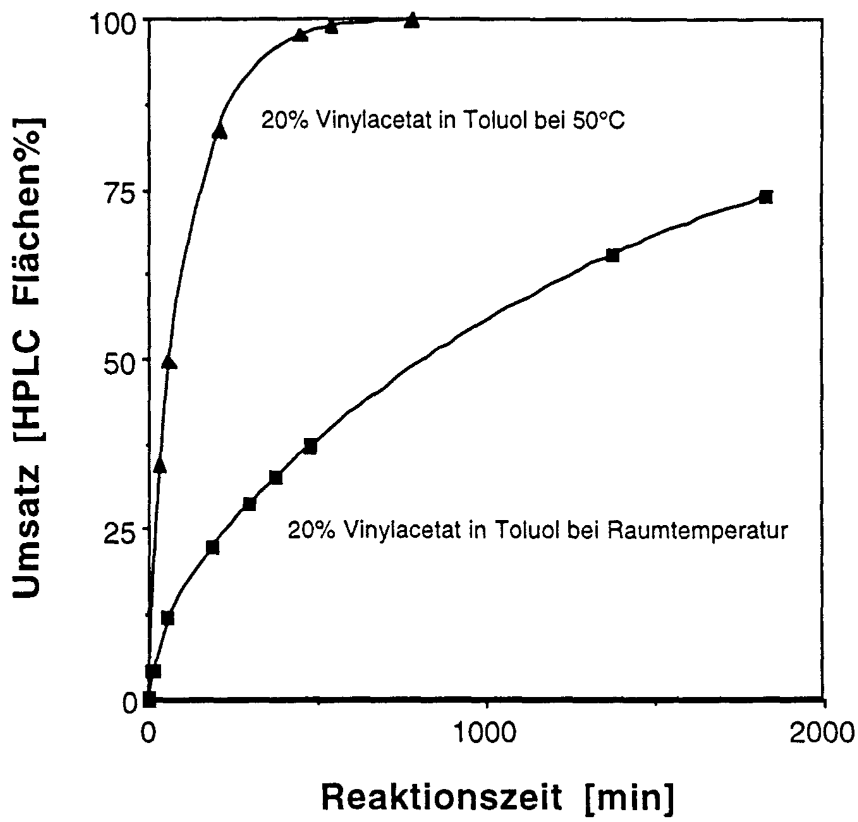


Beispiel 15

Der Einfluss des Lösungsmittels auf die Reaktion wurde mit einem Batch-Verfahrensexperiment geprüft. 48,0 g (157,6 mmol) (11Z,13Z)-7,10-Dihydro-10-hydroxy-retinol wurden in soviel Lösungsmittel, welches 20% (G/V) Vinylacetat enthielt, gelöst, dass das erhaltene Gemisch ein Volumen von 240 ml aufwies. Das homogene Gemisch wurde dann über einen Membranfilter (RC60, 1µm, S&S) filtriert und unter Rühren (100-110 Umdrehungen/Minute) bei 50° gehalten. Die Reaktion wurde durch Zugabe von 3,0 g Lipase PLC (Meito Sangyo) gestartet und mittels HPLC überwacht. Die Ergebnisse sind in der nachfolgenden Tabelle 7 zusammengefasst:

Tabelle 7

| Lösungsmittel | Reaktionszeit (Min.) | Ausbeute (% HPLC) |
|---|---|---|
| Acetonitril | 520 | 97,9 |
| Isopropanol | 420 | 57,4 |
| tert.Butanol | 420 | 79,9 |
| Aceton | 1350 | 99,2 |
| Nitromethan | 480 | 97,1 |
| Methylfuran | 395 | 99,5 |
| Tetrahydrofuran | 1380 | 97,0 |
| Triethylamin | 480 | 92,4 |
| Pyridin | 520 | 68,3 |
| Formaldehyd-dimethylacetal | 270 | 99,6 |
| 1,2-Dimethoxyethan | 480 | 97,3 |
| Diisopropylether | 340 | 99,6 |

Tabelle 7 (fortgesetzt)

| Lösungsmittel | Reaktionszeit (Min.) | Ausbeute (% HPLC) |
|---|---|---|
| Trimethylorthoformiat | 420 | 93,2 |
| tert.Butylmethylether | 480 | 99,3 |
| Toluen | 780 | 99,9 |
| 1,4-Dioxan | 1380 | 96,5 |
| Methylenchlorid | 340 | 99,5 |
| Chloroform | 480 | 96,7 |
| Hexan | 780 | 99,9 |
| Cyclohexan | 480 | 99,6 |
| Tetrachlorkohlenstoff | 480 | 99,3 |

Beispiel 16

Der Einfluss des Acetylierungsmittels auf die Reaktion wurde mit einem Batch-Verfahrensexperiment geprüft. 48,0 g (157,6 mmol) (11Z,13Z)-7,10-Dihydro-10-hydroxy-retinol wurden in soviel Lösungsmittel, welches 20% (G/V) Vinylacetat enthielt, gelöst, dass das erhaltene Gemisch ein Volumen von 240 ml aufwies. Das homogene Gemisch wurde dann über einen Membranfilter (RC60, 1µm, S&S) filtriert und unter Rühren (100-110 Umdrehungen/Minute) bei 50° gehalten. Die Reaktion wurde durch Zugabe von 3,0 g Lipase PLC (Meito Sangyo) gestartet und mittels HPLC überwacht. Die Ergebnisse sind wie folgt tabellarisch (Tabelle 8) und graphisch dargestellt:

Tabelle 8

| Acetylierungsmittel | Reaktionszeit (Min.) | Ausbeute (% HPLC) |
|---|---|---|
| Vinylacetat | 340 | 99,5 |
| Methylacetat | 460 | 40,0 |
| Ethylacetat | 460 | 36,7 |
| Isopropenylacetat | 480 | 6,7 |
| Allylacetat | 480 | 40,2 |

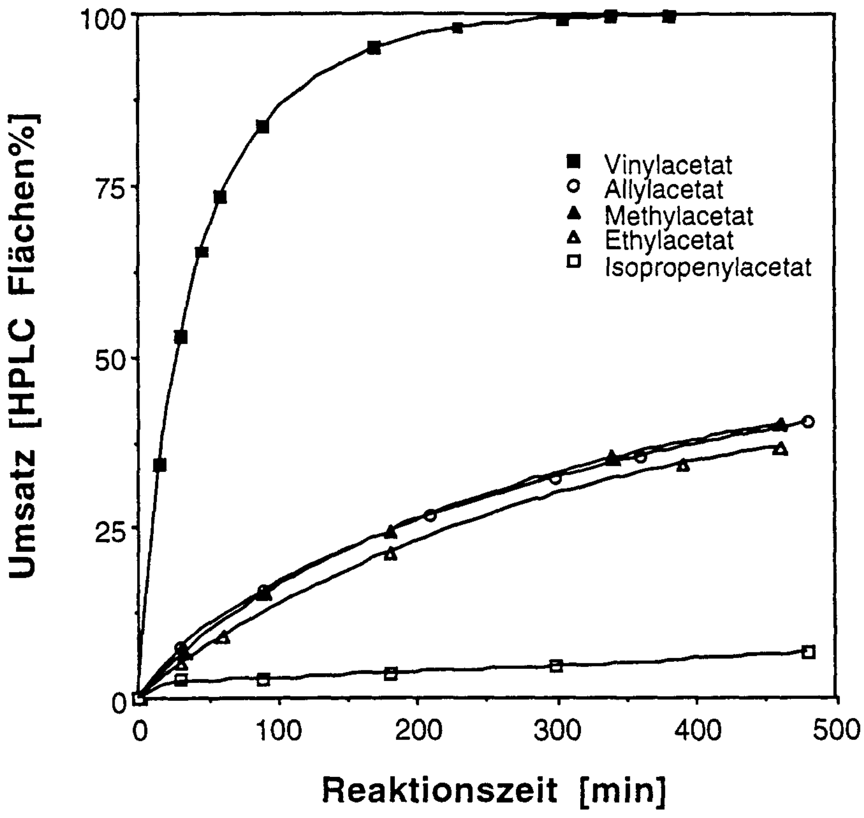


Beispiel 17

Der Einfluss des Acylierungsmittels auf die Reaktion wurde mit einem Batch-Verfahrensexperiment geprüft. 0,5 g (1,64 mmol) (11Z,13Z)-7,10-Dihydro-10-hydroxy-retinol wurden mit 5 ml Vinyl-Ester gelöst bzw. in 5ml Vinyl-Ester suspendiert. Die Reaktion wurde durch Zugabe von 12,5 mg Lipase PLC (Meito Sangyo) gestartet, während 17 Stunden bei Raumtemperatur gerührt (Rollen-Rührer) und mittels HPLC überwacht. Die Ergebnisse sind in der nachfolgenden Tabelle 9 zusammengefasst.

Tabelle 9

| Vinyl-Ester | Ausbeute (% HPLC) |
|---|---|
| Vinylacetat | 87,5 |
| Vinylpropionat | 47,6 |
| Vinylbutyrat | 81,2 |
| Vinylcrotonat | 14,8 |
| Vinyllaurat | 21,6 |

Beispiel 18

Der Einfluss des Acylierungsmittels auf die Reaktion wurde mit einem Batch-Verfahrensexperiment geprüft. 0,5 g (1,64 mmol) (11Z,13Z)-7,10-Dihydro-10-hydroxy-retinol wurden mit 5 ml Vinyl-Ester gelöst bzw. in 5ml Vinyl-Ester suspendiert. Die Reaktion wurde durch Zugabe von 12,5 mg Lipase Chirazyme® L-2 (Boehringer Mannheim GmbH, ex Novozym® 435 von Novo Nordisk) gestartet, während 17 Stunden bei Raumtemperatur gerührt (Rollen-Rührer) und mittels HPLC überwacht. Die Ergebnisse sind in der nachfolgenden Tabelle 10 zusammengefasst.

Tabelle 10

| Vinyl-Ester | Ausbeute (% HPLC) |
|---|---|
| Vinylacetat | 96,3 |
| Vinylpropionat | 77,2 |
| Vinylbutyrat | 99,0 |
| Vinylcrotonat | 2,8 |
| Vinyllaurat | 36,3 |

**Patentansprüche**

1. Verfahren zur Herstellung eines (11Z,13Z)-7,10-Dihydro-10-hydroxy-retinylacylats der Formel

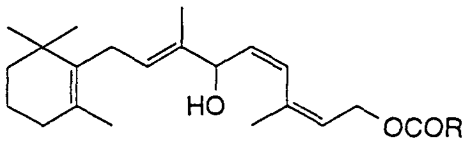

I

worin R eine $C_{1-23}$-Alkylgruppe oder eine 1 bis 3 Doppelbindungen aufweisende $C_{2-23}$-Alkenylgruppe bedeutet,
dadurch gekennzeichnet, dass (11Z,13Z)-7,10-Dihydro-10-hydroxy-retinol der Formel

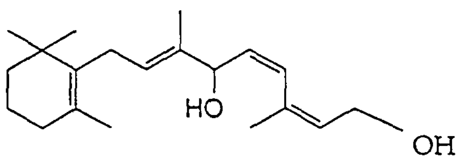

II

in einem organischen Lösungsmittel in Gegenwart eines Acylierungsmittels, welches ein Alkyl- oder Alkenylacylat ist, mit einer in Suspension vorliegenden Lipase selektiv monoacyliert wird.

2. Verfahren nach Anspruch 1, dadurch gekennzeichnet, dass es sich bei der Lipase um Lipase PL aus *Alcaligenes sp.* , Lipase PLC, Lipase PLG, Lipase MY-30 aus *Candida cylindracea* (*Candida rugosa*), Lipozyme® IM-20 aus *Mucor miehei* (*Rhizomucor miehei*), Lipase CE-5 aus *Humicola lanuginosa* oder Lipase G aus *Penicillium cyclopium* handelt.

3. Verfahren nach Anspruch 1, dadurch gekennzeichnet, dass es sich bei der Lipase um Chirazyme® L-2 aus *Candida antarctica* handelt.

4. Verfahren nach einem der Ansprüche 1 bis 3, dadurch gekennzeichnet, dass R der Formel I Methyl bedeutet, wobei (11Z,13Z)-7,10-Dihydro-10-hydroxy-retinol der Formel II selektiv monoacetyliert wird.

5. Verfahren nach einem der Ansprüche 1 bis 4, dadurch gekennzeichnet, dass der Anteil an Lipase bis zu 20 Gew.-% bezogen auf (11Z,13Z)-7,10-Dihydro-10-hydroxy-retinol beträgt.

6. Verfahren nach Anspruch 5, dadurch gekennzeichnet, dass der Anteil an Lipase zwischen etwa 0,1 Gew.-% und etwa 10 Gew.-% liegt.

7. Verfahren nach einem der Ansprüche 1 bis 6, dadurch gekennzeichnet, dass die Lipase in immobilisierter Form vorliegt.

**8.** Verfahren nach Anspruch 7, dadurch gekennzeichnet, dass die Lipase in Gegenwart eines Gallensalzes immobilisiert wurde.

**9.** Verfahren nach einem der Ansprüche 1 bis 8, dadurch gekennzeichnet, dass die Umsetzung unter Inertgasatmosphäre und unter Lichtausschluss und/oder in Gegenwart eines Radikalfängers durchgeführt wird.

**10.** Verfahren nach einem der Ansprüche 1 bis 9, dadurch gekennzeichnet, dass als Lösungsmittel ein aliphatischer Kohlenwasserstoff mit 5-8 Kohlenstoffatomen, ein cyclischer Kohlenwasserstoff mit 6-10 Kohlenstoffatomen, ein chlorierter aliphatischer Kohlenwasserstoff, ein nitrosubstituierter aliphatischer Kohlenwasserstoff, ein aromatischer Kohlenwasserstoff, ein aliphatischer Ether, ein cyclischer Ether, ein aliphatischer Ester, ein aliphatisches Keton, ein aliphatisches Nitril, ein aliphatisches Amin, ein aliphatisches Acetal oder ein Gemisch solcher Lösungsmittel eingesetzt wird.

**11.** Verfahren nach Anspruch 10, dadurch gekennzeichnet, dass als Lösungsmittel n-Hexan, Cyclohexan, Methylenchlorid, Tetrachlorkohlenstoff, Toluen, Diisopropylether, Tetrahydrofuran, Methylfuran, Vinylacetat, Vinylpropionat oder Formaldehyd-dimethylacetal ,oder ein Gemisch solcher Lösungsmittel eingesetzt wird.

**12.** Verfahren nach einem der Ansprüche 1 bis 11, dadurch gekennzeichnet, dass als Acetylierungsmittel Ethylacetat, Butylacetat oder Vinylacetat eingesetzt wird.

**13.** Verfahren nach einem der Ansprüche 1 bis 12, dadurch gekennzeichnet, dass die Konzentration an (11Z,13Z)-7,10-Dihydro-10-hydroxyretinol 10% bis 50% (G/V) beträgt.

**14.** Verfahren nach einem der Ansprüche 1 bis 13, dadurch gekennzeichnet, dass die Reaktionstemperatur zwischen etwa 10°C und der Rückflusstemperatur des Reaktionsgemisches. liegt.

**15.** Verfahren nach einem der Ansprüche 1 bis 14, dadurch gekennzeichnet, dass der Reaktionslösung eine geringe Menge (<0.2% ) an Wasser oder einer schwach basischen Lösung zugegeben wird.

**16.** Verfahren nach einem der Ansprüche 1 bis 15, dadurch gekennzeichnet, dass das (11Z,13Z)-7,10-Dihydro-10-hydroxy-retinol zur Entfernung von schädlichen Verunreinigungen über einen Filtrierhilfsstoff filtriert oder mit wässriger Ethylendiamin-tetraessigsäure-Lösung bei einem pH-Wert von etwa 8 gewaschen wird.

**17.** Verfahren nach einem der Ansprüche 1 bis 16, dadurch gekennzeichnet, dass das Verfahren als wiederholtes Batch-Verfahren oder als kontinuierliches Verfahren durchgeführt wird.

**18.** Verfahren nach einem der Ansprüche 1 bis 17, dadurch gekennzeichnet, dass das erhaltene (11Z,13Z)-7,10-Dihydro-10-hydroxy-retinyl-acylat ,durch Wasserabspaltung unter gleichzeitiger cis-trans-Isomerisierung in das entsprechende Vitamin A-acylat übergeführt wird.

## Claims

**1.** A process for the manufacture of a (11Z,13Z)-7,10-dihydro-10-hydroxy-retinyl acylate of the formula

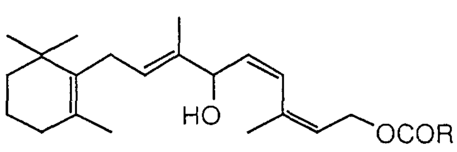

I

wherein R signifies a $C_{1-23}$-alkyl group or a $C_{2-23}$-alkenyl group containing 1 to 3 double bonds, which process comprises selectively monoacylating (11Z,13Z)-7,10-dihydro-10-hydroxy-retinol of the formula

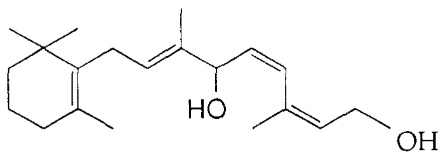

II

in an organic solvent in the presence of an acylating agent, which is an alkyl or alkenyl acetate,with a lipase which is present in suspension.

2. A process according to claim 1, wherein the lipase is lipase PL from *Alcaligenes sp*., lipase PLC, lipase PLG, lipase MY-30 from *Candida cylindracea*, (*Candida rugosa*), Lipozyme® IM-20 from *Mucor miehei* (*Rhizomucor miehei*), lipase CE-5 from *Humicola lanuginosa* or lipase G from *Penicillium cyclopium*.

3. A process according to claim 1, wherein the lipase is Chirazyme® L-2 from *Candida antarctica.*

4. A process according to any one of claims 1 to 3, wherein R in formula I signifies methyl, whereby (11Z,13Z)-7,10-dihydro-10-hydroxy-retinol of formula II is selectively monoacetylated.

5. A process according to any one of claims 1 to 4, wherein the amount of lipase is up to 20% wt.% based on (11Z, 13Z)-7,10-dihydro-10-hydroxy-retinol.

6. A process according to claim 5, wherein the amount of lipase is between about 0.1 wt.% and about 10 wt.%.

7. A process according to any one of claims 1 to 6, wherein the lipase is present in immobilized form.

8. A process according to claim 7, wherein the lipase has been immobilized in the presence of a cholanic salt.

9. A process according to any one of claims 1 to 8, wherein the reaction is carried out under an inert gas atmosphere and with the exclusion of light and/or in the presence of a radical scavenger.

10. A process according to any one of claims 1 to 9, wherein an aliphatic hydrocarbon with 5-8 carbon atoms, a cyclic hydrocarbon with 6-10 carbon atoms, a chlorinated aliphatic hydrocarbon, a nitro-substituted aliphatic hydrocarbon, an aromatic hydrocarbon, an aliphatic ether, a cyclic ether, an aliphatic ester, an aliphatic ketone, an aliphatic nitrile, an aliphatic amine, an aliphatic acetal or a mixture of such solvents is used as the solvent.

11. A process according to claim 10, wherein n-hexane, cyclohexane, methylene chloride, carbon tetrachloride, toluene, diisopropyl ether, tetrahydrofuran, methylfuran, vinyl acetate, vinyl propionate or formaldehyde dimethyl acetal or a mixture of such solvents is used.

12. A process according to any one of claims 1 to 11, wherein ethyl acetate, butyl acetate or vinyl acetate is used as the acetylating agent.

13. A process according to any one of claims 1 to 12, wherein the concentration of (11Z,13Z)-7,10-dihydro-10-hydroxy-retinol is 10% to 50% (wt./vol.).

14. A process according to any one of claims 1 to 13, wherein the reaction temperature lies between about 10°C and the reflux temperature of the reaction mixture.

15. A process according to any one of claims 1 to 14, wherein a small amount (<0.2%) of water or of a weak basic solution is added to the reaction solution.

16. A process according to any one of claims 1 to 15, wherein the (11Z,13Z)-7,10-dihydro-10-hydroxy-retinol is filtered over a filter aid or washed with aqueous ethylenediaminetetraacetic acid solution having a pH value of about 8 in order to remove harmful impurities.

17. A process according to any one of claims 1 to 16, wherein the process is carried out as a repeated batch process

or as a continuous process.

**18.** A process according to any one of claims 1 to 17, wherein the obtained (11Z,13Z)-7,10-dihydro-10-hydroxy-retinyl acylate is converted into the corresponding vitamin A acylate by elimination of water with simultaneous trans isomerization.

**Revendications**

**1.** Procédé pour la préparation d'un acylate de (11Z,13Z)-7,10-dihydro-10-hydroxyrétinyle de formule :

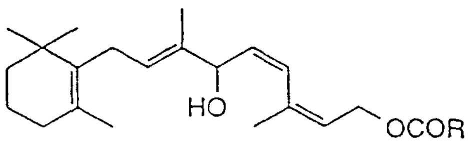

(I)

où R représente un groupe alkyle en $C_1$-$C_{23}$ ou un groupe alcényle en $C_2$-$C_{23}$ présentant 1 à 3 doubles liaisons, caractérisé en ce que l'on monoacyle, de façon sélective, le (11Z,13Z)-7,10-dihydro-10-hydroxyrétinol de formule :

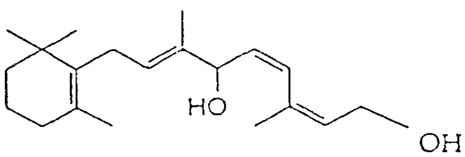

(II)

dans un solvant organique en présence d'un agent d'acylation, ledit agent d'acylation étant un acylate d'alkyle ou un acylate d'alcényle, avec une lipase se trouvant en suspension.

**2.** Procédé selon la revendication 1, caractérisé en ce que la lipase est la lipase PL de Alcaligenes sp., la lipase PLC, la lipase PLG, la lipase MY-30 de Candida cylindracea (Candida rugosa), la lipase Lipozyme® IM-20 de Mucor miehei (Rhizomucor miehei), la lipase LE-5 de Humicola lanuginosa ou la lipase G de Pénicillium cyclopium.

**3.** Procédé selon la revendication 1, caractérisé en ce que la lipase est la lipase Chirazyme® L-2 de Candida antarctica.

**4.** Procédé selon l'une des revendications 1 à 3, caractérisé en ce que R dans la formule I représente le méthyle, le (11Z,13Z)-7,10-dihydro-10-hydroxyrétinol de formule II étant monoacétylé de façon sélective.

**5.** Procédé selon l'une des revendications 1 à 4, caractérisé en ce que la proportion de lipase va jusqu'à 25 % en poids rapportée au (11Z,13Z)-7,10-dihydro-10-hydroxyrétinol.

**6.** Procédé selon la revendication 5, caractérisé en ce que la proportion de lipase est comprise entre environ 0,1 % en poids et environ 10 % en poids.

**7.** Procédé selon l'une des revendications 1 à 6, caractérisé en ce que la lipase se présente sous forme immobilisée.

**8.** Procédé selon la revendication 7, caractérisé en ce que la lipase est immobilisée en présence d'un sel biliaire.

**9.** Procédé selon l'une des revendications 1 à 8, caractérisé en ce que la réaction est effectuée sous une atmosphère de gaz inerte et à l'abri de la lumière et/ou en présence d'un fixateur de radicaux.

**10.** Procédé selon l'une des revendications 1 à 9, caractérisé en ce que l'on utilise, comme solvant, un hydrocarbure aliphatique comportant 5 à 8 atomes de carbone, un hydrocarbure cyclique comportant 6 à 10 atomes de carbone, un hydrocarbure aliphatique chloré, un hydrocarbure aliphatique substitué par des groupes nitro, un hydrocarbure aromatique, un éther aliphatique, un éther cyclique, un ester aliphatique, une cétone aliphatique, un nitrile aliphatique, une amine aliphatique, un acétal aliphatique ou un mélange de ces solvants.

**11.** Procédé selon la revendication 10, caractérisé en ce que l'on utilise, comme solvant, le n-hexane, le cyclohexane, le chlorure de méthylène, le tétrachlorure de carbone, le toluène, le diisopropyléther, le tétrahydrofuranne, le méthylfuranne, l'acétate de vinyle, le propionate de vinyle ou le formaldéhydediméthylacétal ou un mélange de ces solvants.

**12.** Procédé selon l'une des revendications 1 à 11, caractérisé en ce que l'on utilise, comme agent d'acétylation, l'acétate d'éthyle, l'acétate de butyle ou l'acétate de vinyle.

**13.** Procédé selon l'une des revendications 1 à 12, caractérisé en ce que la concentration en (11Z,13Z)-7,10-dihydro-10-hydroxyrétino) est comprise entre 10 % et 50 % (p/v).

**14.** Procédé selon l'une des revendications 1 à 13, caractérisé en ce que la température réactionnelle est comprise entre environ 10°C et la température de reflux du mélange réactionnel.

**15.** Procédé selon l'une des revendications 1 à 14, caractérisé en ce que l'on additionne à la solution réactionnelle une faible quantité (< 0,2 %) d'eau ou d'une solution faiblement basique.

**16.** Procédé selon l'une des revendications 1 à 15, caractérisé en ce que l'on filtre le (11Z,13Z)-7,10-dihydro-10-hydroxyrétinol sur un adjuvant de filtration pour éliminer les impuretés nuisibles ou en ce que l'on lave avec une solution aqueuse éthylènediamine-acide tétraacétique à un pH d'environ 8.

**17.** Procédé selon l'une des revendications 1 à 16, caractérisé en ce que le procédé est exécuté sous la forme d'un procédé discontinu ou sous la forme d'un procédé continu.

**18.** Procédé selon l'une des revendications 1 à 17, caractérisé en ce que l'acylate de (11Z,13Z)-7,10-dihydro-10-hydroxyrétinyle obtenu est transformé par élimination d'eau avec isomérisation cis-trans simultanée en acylate de vitamine A correspondant.